# EUROPEAN PATENT APPLICATION

(11) **EP 4 385 537 A1**
(43) Date of publication of application: **19.06.2024**
(21) Application number: 21953851.9
(22) Date of filing: 24.08.2021
(51) Int. Cl.: A61M 1/38

(54) **LOW VOLUME PLASMA EXCHANGE DEVICE**

(30) Priority: 16.08.2021 CN 202110938109
(71) Applicant: Scinomed (Shanghai) Bio-Tech Co., Ltd., Shanghai 200126 (CN); Song, HongBiao, Shanghai 200126 (CN); Wang, Xiao, Shanghai 200126 (CN)
(72) Inventor: SONG, HongBiao, Shanghai 200126 (CN); WANG, Xiao, Shanghai 200126 (CN)
(74) Representative: Ipside
(86) International application number: PCT/CN2021/114286
(87) International publication number: WO 2023/019610

(57) **Abstract**

The disclosure describes a low-capacity plasma exchange device, comprising a chassis assembly, wherein a centrifuge component is arranged on the inner side of the chassis assembly, and a medication pump, an anticoagulant pump, and a blood drawing pump are arranged on one side of the top of the chassis assembly relative to the centrifuge component, and a first air detector component is arranged on one side of both the medication pump and the blood drawing pump; in the disclosure, the low-capacity plasma exchange device first collects a certain amount of plasma from the patient's body, then mixes the treatment medication into the blood cells after the removal of plasma and reinfuses them back into the patient's body, achieving the effect of exchange treatment, significantly improving the treatment effect, and solving the problem of poor treatment effects of oral medication and infusion therapy in the existing technology.

## Description

### Technical Field

The present invention belongs to the field of medical devices, specifically relating to a low-capacity plasma exchange device.

### Background Art

In clinical medical applications, some diseases require patients to undergo long-term and regular treatment. In the treatment process, the existing methods generally include oral medication and intravenous infusion. These methods have problems with poor treatment effects in practical use and cannot effectively improve the patient's condition, presenting certain disadvantages. Therefore, this disclosure proposes a low-capacity plasma exchange device.

### Summary of the Invention

The purpose of this disclosure is to provide a low-capacity plasma exchange device to solve the problems mentioned in the background art.

To achieve the above objectives, the present disclosure provides the following technical solutions: a low-capacity plasma exchange device, including a chassis assembly, with a centrifuge component arranged inside the chassis assembly. On one side of the top of the chassis assembly, relative to the centrifuge component, there are a medication pump, an anticoagulant pump, and a blood drawing pump. On one side of both the medication pump and the blood drawing pump, there is a first air detector component. On the other side of the top of the chassis assembly, relative to the centrifuge component, there is a wire sensor component, and on one side of the wire sensor component, there are two first solenoid valves. At the rear of the top of the chassis assembly, relative to the centrifuge component, there is a display screen. On the front surface of the chassis assembly, there are two second air detector components, and above the second air detector components, there is a patient pressure detector component. The top of the chassis assembly also has an inclined surface, on which there is a second solenoid valve and a scale component.

Preferably, the medication pump includes a base plate, a rotating seat, and flexible wheels. The base plate is fixed on the top of the chassis assembly, the rotating seat is rotatably arranged on the inside of the base plate, and three flexible wheels are rotatably mounted on the top of the rotating seat.

Preferably, on the top of the chassis assembly, relative to the two sides of the display screen, there are a first hanger and a second hanger assembly, and on the two side surfaces of the chassis assembly, there are a first side display component and a second side display component.

Preferably, on one side surface of the chassis assembly, relative to the bottom of the first side display component, there is a card reader and barcode scanner component, and on the other side surface of the chassis assembly, relative to the bottom of the second side display component, there is a drip sensor.

Preferably, on the front surface of the chassis assembly, there is a front panel alarm indicator, and the front panel alarm indicator is located on one side of the second air detector component.

Preferably, inside the chassis assembly, there are a power supply component, a control box assembly, a rear adapter module, and an inflation component. The power supply component and the control box assembly are respectively arranged on the two inner sides of the chassis assembly, and the rear adapter module and the inflation component are arranged on one side of the control box assembly.

Compared with the existing technology, the beneficial effects of the present disclosure are: the low-capacity plasma exchange device of the present disclosure first collects a certain amount of plasma from the patient's body, then mixes the treatment medication into the blood cells after the removal of plasma and reinfuses them into the patient's body, achieving the effect of exchange treatment and significantly improving the treatment effect. It solves the problem of poor treatment effects of oral medication and infusion in existing technology. Moreover, when the patient's blood is drawn out, it stimulates the patient's hematopoietic system and organs such as the heart, and when the blood cells with medication are subsequently reinfused into the patient's body, it will again stimulate the hematopoietic system and organs such as the heart, thereby improving the treatment effect on the patient's disease and improving the condition.

### Brief Description of the Drawings

FIG. 1 is a schematic structural diagram of the present disclosure;
FIG. 2 is a rear view of the present disclosure with the rear cover removed;
FIG. 3 is a top view of the medication pump of the present disclosure;
FIG. 4 is a schematic diagram of the principle of the present disclosure;

List of reference numerals: 1 - chassis assembly; 2 - display screen; 3 - first hanger; 4 - first solenoid valve; 5 - wire sensor component; 6 - first side display component; 7 - scale component; 8 - card reader and barcode scanner component; 9 - second hanger assembly; 10 - medication pump; 101 - base plate; 102 - rotating seat; 103 - flexible wheel; 11 - blood drawing pump; 12 - first air detector component; 13 - second solenoid valve; 14 - centrifuge component; 15 - patient pressure detector component; 16 - second air detector component; 17 - second side display component; 18 - drip sensor; 19 - power supply component; 20 - control box assembly; 21 - rear adapter module; 22 - inflation component; 23 - front panel alarm indicator; 24 - anticoagulant pump.

### Detailed Description of Embodiments

The following will combine the drawings in the embodiments of the present disclosure to clearly and completely describe the technical solutions in the embodiments of the present disclosure. Obviously, the described embodiments are only a part of the embodiments of the present disclosure, not all of them. Based on the embodiments in this disclosure, all other embodiments obtained by those skilled in the art without creative efforts fall within the scope of protection of this disclosure.

### Embodiment

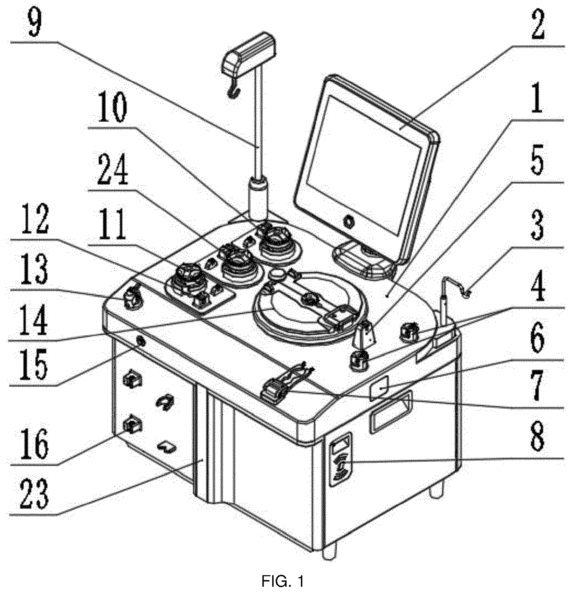

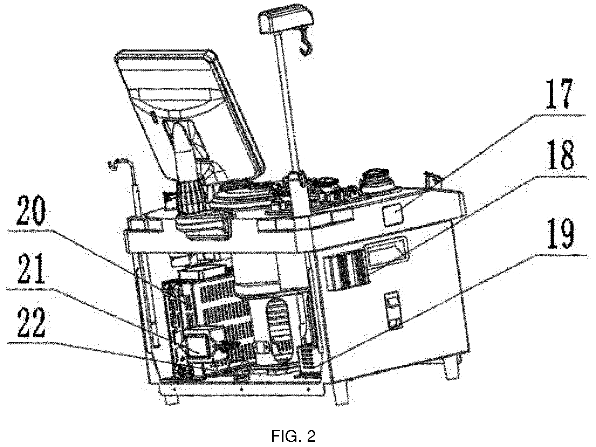

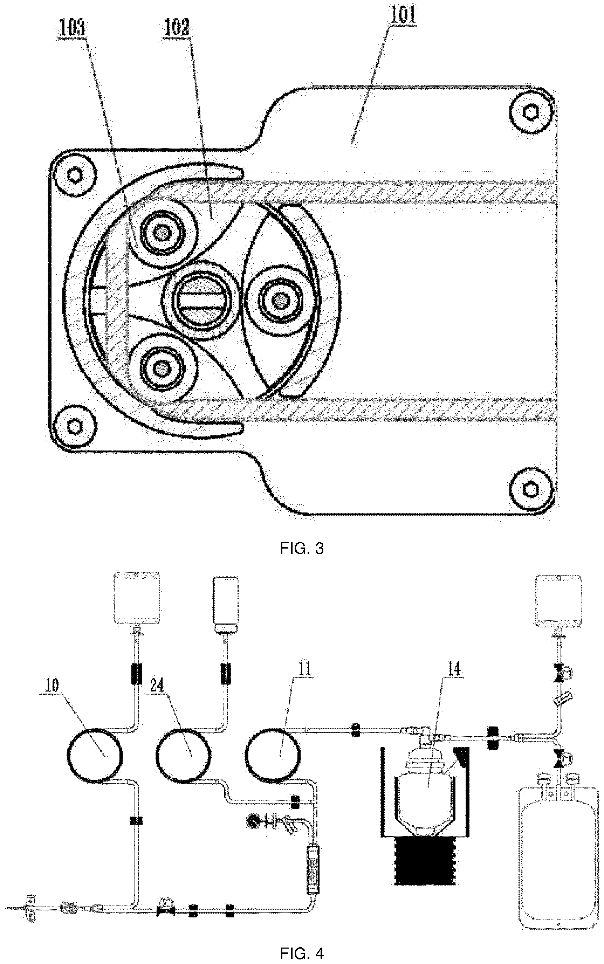

Please refer to FIGS. 1 to 4, the present disclosure provides a technical solution: a low-capacity plasma exchange device, including a chassis assembly 1, with a centrifuge component 14 arranged inside the chassis assembly 1. On one side of the top of the chassis assembly 1, relative to the centrifuge component 14, there are a medication pump 10, an anticoagulant pump 24, and a blood drawing pump 11. On one side of both the medication pump 10 and the blood drawing pump 11, there is a first air detector component 12. On the other side of the top of the chassis assembly 1, relative to the centrifuge component 14, there is a wire sensor component 5, and on one side of the wire sensor component 5, there are two first solenoid valves 4. At the rear of the top of the chassis assembly 1, relative to the centrifuge component 14, there is a display screen 2. On the front surface of the chassis assembly 1, there are two second air detector components 16, and above the second air detector components 16, there is a patient pressure detector component 15. The top of the chassis assembly 1 also has an inclined surface, on which there is a second solenoid valve 13 and a scale component 7. The scale component 7 includes a weight sensor and a foldable weighing cantilever, with the foldable weighing cantilever fixed on the chassis assembly 1, and the weight sensor located within the main body of the device with the foldable weighing cantilever, saving space and also preventing damage to the weight sensor during transportation and handling.

In this embodiment, preferably, the medication pump 10 includes a base plate 101, a rotating seat 102, and flexible wheels 103. The base plate 101 is fixed on the top of the chassis assembly 1, and the rotating seat 102 is rotatably arranged on the inside of the base plate 101 through a brushless servo motor. A planetary reducer connected to the brushless servo motor is installed below the base plate 101, making the operation of the medication pump 10 very slow, thus making the medication more precise during the reinfusion process. The rotating seat 102 has three flexible wheels 103 mounted on its top, adopting a multi-wheel structure design, which has high flow precision.

In this embodiment, preferably, on the top of the chassis assembly 1, relative to the two sides of the display screen 2, there are a first hanger 3 and a second hanger assembly 9, and on the two side surfaces of the chassis assembly 1, there are a first side display component 6 and a second side display component 17.

In this embodiment, preferably, on one side surface of the chassis assembly 1, relative to the bottom of the first side display component 6, there is a card reader and barcode scanner component 8. The card reader and barcode scanner component 8 uses an optical sensor to read barcodes and QR codes on disposable packaging, and then, according to the requirements of the plasma center, these pieces of information can be processed. On the other side surface of the chassis assembly 1, relative to the bottom of the second side display component 17, there is a drip sensor 18. The drip sensor 18 calculates the actual flow rate of the liquid by calculating the volume of the infusion liquid detected per unit time, ensuring that the infusion speed is more precise and controllable, making the infusion process safer, and ensuring the speed and precision of infusing anticoagulants, human albumin, and IGIV.

In this embodiment, preferably, on the front surface of the chassis assembly 1, there is a front panel alarm indicator 23, and the front panel alarm indicator 23 is located on one side of the second air detector component 16.

In this embodiment, preferably, inside the chassis assembly 1, there are a power supply component 19, a control box assembly 20, a rear adapter module 21, and an inflation component 22. The power supply component 19 and the control box assembly 20 are respectively arranged on the two inner sides of the chassis assembly 1, and the rear adapter module 21 and the inflation component 22 are arranged on one side of the control box assembly 20.

The working principle and usage process of the disclosure: When using the low-capacity plasma exchange device, first connect the blood drawing pump 11 to the transfusion tube to collect blood from the patient's body. During the collection process, the anticoagulant pump 24 will inject anticoagulant into the transfusion tube to ensure that the blood does not coagulate during extraction. The patient's blood will be drawn into the centrifuge component 14 under the action of the blood drawing pump 11, completing the separation of plasma and red blood cells. When the collected plasma reaches the target value, the blood drawing pump 11 is reversed to reinfuse the blood cells, after plasma removal, directly back into the patient's body without passing through the centrifuge component 14. At the same time, during reinfusion, the medication pump 10 is started, and the treatment medication is added to the transfusion tube through the pipeline. The planetary reducer below the medication pump 10 will make the operation of the medication pump 10 slower through the internal planetary gears, thus making the medication more precise during the reinfusion process. The reinfused blood cells will be mixed with the treatment medication and enter the patient's body, achieving the treatment effect. Meanwhile, when the patient's blood is drawn out, it stimulates the patient's hematopoietic system and organs such as the heart, and when the blood cells with medication are subsequently reinfused into the patient's body, it will again stimulate the hematopoietic system and organs such as the heart, thereby improving the treatment effect on the patient's disease and improving the condition.

Although the embodiments of the present disclosure have been shown and described, it can be understood by those skilled in the art that various changes, modifications, substitutions, and variations can be made to these embodiments without departing from the principles and spirit of the disclosure. The scope of the disclosure is defined by the attached claims and their equivalents.

## Claims

1. A low-capacity plasma exchange device, comprising a chassis assembly (1), **characterized in that**: the inside of the chassis assembly (1) is equipped with a centrifuge component (14), and the top of the chassis assembly (1) on one side relative to the centrifuge component (14) is equipped with a medication pump (10), an anticoagulant pump (24), and a blood drawing pump (11), each side of the medication pump (10), anticoagulant pump (24), and blood drawing pump (11) is equipped with a first air detector component (12), the top of the chassis assembly (1) on the other side relative to the centrifuge component (14) is equipped with a wire sensor component (5), and one side of the wire sensor component (5) is equipped with two first solenoid valves (4), the top of the chassis assembly (1) at the rear relative to the centrifuge component (14) is equipped with a display screen (2), the front surface of the chassis assembly (1) is equipped with two second air detector components (16), and above the second air detector components (16) is equipped with a patient pressure detector component (15), the top of the chassis assembly (1) is also equipped with an inclined surface, on which are equipped a second solenoid valve (13) and a scale component (7).

2. The low-capacity plasma exchange device according to claim 1, **characterized in that**: the medication pump (10) includes a base plate (101), a rotating seat (102), and flexible wheels (103), the base plate (101) is fixed to the top of the chassis assembly (1), the inside of the base plate (101) is rotatably equipped with the rotating seat (102), and the top of the rotating seat (102) is rotatably equipped with three flexible wheels (103).

3. The low-capacity plasma exchange device according to claim 1, **characterized in that**: the top of the chassis assembly (1) on both sides relative to the display screen (2) is respectively equipped with a first hanger (3) and a second hanger assembly (9), and the two side surfaces of the chassis assembly (1) are respectively equipped with a first side display component (6) and a second side display component (17).

4. The low-capacity plasma exchange device according to claim 1, **characterized in that**: one side surface of the chassis assembly (1) at the bottom relative to the first side display component (6) is equipped with a card reader and barcode scanner component (8), and the other side surface of the chassis assembly (1) at the bottom relative to the second side display component (17) is equipped with a drip sensor (18).

5. The low-capacity plasma exchange device according to claim 1, **characterized in that**: the front surface of the chassis assembly (1) is equipped with a front panel alarm indicator (23), and the front panel alarm indicator (23) is located on one side of the second air detector component (16).

6. The low-capacity plasma exchange device according to claim 1, **characterized in that**: the inside of the chassis assembly (1) is equipped with a power supply component (19), a control box assembly (20), a rear adapter module (21), and an inflation component (22), the power supply component (19) and the control box assembly (20) are respectively located on the two inner sides of the chassis assembly (1), and the rear adapter module (21) and the inflation component (22) are located on one side of the control box assembly (20).
